(19) Europäisches Patentamt / European Patent Office / Office européen des brevets

(11) EP 2 070 946 B1

(12) EUROPÄISCHE PATENTSCHRIFT

(45) Veröffentlichungstag und Bekanntmachung des Hinweises auf die Patenterteilung:
29.05.2013 Patentblatt 2013/22

(51) Int Cl.:
*C07K 14/605* (2006.01)        *C07K 14/575* (2006.01)
*A61K 38/16* (2006.01)

(21) Anmeldenummer: 08020703.8

(22) Anmeldetag: 26.08.2005

(54) **Erfindung betreffend GLP-1 und Exendin**

Invention affecting GLP-1 and Exendin

Invention concernant GLP-1 et l'exendine

(84) Benannte Vertragsstaaten:
AT BE BG CH CY CZ DE DK EE ES FI FR GB GR HU IE IS IT LI LT LU LV MC NL PL PT RO SE SI SK TR

(30) Priorität: 03.09.2004 DE 102004043153

(43) Veröffentlichungstag der Anmeldung:
17.06.2009 Patentblatt 2009/25

(62) Dokumentnummer(n) der früheren Anmeldung(en) nach Art. 76 EPÜ:
05778889.5 / 1 784 422

(73) Patentinhaber: Philipps-Universität Marburg
35037 Marburg (DE)

(72) Erfinder:
• Béhé, Martin, Dr.
79016 Freiburg (DE)
• Gotthardt, Martin, Dr.
6500 HB Nijmegen (NL)
• Behr, Thomas, Prof. Dr.
35043 Marburg (DE)
• Göke, Burkhard J., Prof. Dr.
82131 Gauting (DE)

(74) Vertreter: Buchhold, Jürgen
Patentanwälte
Olbricht Buchhold Keulertz Partnerschaft
Bettinastrasse 53-55
60325 Frankfurt am Main (DE)

(56) Entgegenhaltungen:
WO-A-00/69900          WO-A-01/04156
WO-A-91/01144          WO-A-97/46584
US-A1- 2003 073 626    US-A1- 2003 232 761

• ENG J ET AL: "PURIFICATION AND STRUCTURE OF EXENDIN-3, A NEW PANCREATIC SECRETAGOGUE ISOLATED FROM HELODERMA HORRIDUM VENOM" JOURNAL OF BIOLOGICAL CHEMISTRY, AMERICAN SOCIETY OF BIOLOCHEMICAL BIOLOGISTS, BIRMINGHAM, US, Bd. 265, Nr. 33, 25. November 1990 (1990-11-25), Seiten 20259-20262, XP002045292 ISSN: 0021-9258

• STENNICKE H R ET AL: "C-terminal incorporation of fluorogenic and affinity labels using wild-type and mutagenized carboxypeptidase Y" ANALYTICAL BIOCHEMISTRY, ACADEMIC PRESS, SAN DIEGO, CA, US, Bd. 248, Nr. 1, 15. Mai 1997 (1997-05-15), Seiten 141-148, XP002298918 ISSN: 0003-2697

EP 2 070 946 B1

**Beschreibung**

**Gebietes der Erfindung**

Bei der Lokalisationsdiagnostik von gastroenteropankreatischen neuroendokrinen Tumoren wird als wichtigste diagnostische Methode neben dem Ultraschall die Somatostatin Rezeptor Szintigraphie (SRS) eingesetzt. Das Prinzip ist hier die spezifische Darstellung von Tumoren mit Hilfe von radioaktiv markierten Peptiden, die in die Tumorzellen aufgenommen werden. Sodann kann mit Hilfe von Gammakameras die Anreicherung der Radioaktivität im Tumorgewebe bildlich nachgewiesen werden. Wenn ein Tumortyp einen für die SRS erforderlichen Rezeptor z.B. für das Somatostatin-Analogon Octreotide® besitzt, ist ein Nachweis dieser Tumoren problemlos möglich. Werden entsprechende Rezeptoren jedoch nicht exprimiert, entziehen sie sich dem szintigraphischen Nachweis. Neben der Lokalisationsdiagnostik ermöglichen radioakiv markierte Peptide aber auch einen Ansatz zur Tumorbehandlung, indem man durch Markierung von Somatostatin-Analoga z.B. Octreotide® mit einem geeigneten Radionuklid ($\alpha$- oder $\beta$-Strahler) eine spezifische rezeptorgerichtete Radiopeptidtherapie durchführen kann. Da die entsprechenden Radionuklide chemisch so vom Peptid gebunden werden (z.B. über Komplexierung mit einem zuvor an das Peptid gebundenen Metallchelator), dass sie zwar in die Tumorzelle aufgenommen aber nicht mehr ausgeschleust werden können, ergibt sich eine hohe spezifische Anreicherung im Tumorgewebe.

Allerdings exprimieren eine ganze Reihe von neuroendokrinen Tumoren (NET) darunter Insulinome und kleinzellige Bronchialkarzinome nicht die erforderlichen Subtypen des Somatostatinrezeptors, die für die SRS oder die Radiopeptidetheraphie mit dem Somatostatin-Analogon Octreotide® notwendig sind. Insbesondere Insulinome entziehen sich in einem erheblichen Prozentsatz der szintigraphischen Diagnostik. Auch bei kleinzelligen Bronchialkarzinomen stellt die SRS kein geeignetes Verfahren dar, da zwar die Primärtumore häufig darstellbar sind, die Metastasen jedoch nicht, da sie die Rezeptorexpression verloren haben. Somit sind sie auch keiner Radiopeptidtherapie zugänglich, die ein interessantes zusätzliches oder alternatives Therapieverfahren darstellt. Daher besteht der Bedarf an einem geeigneten Peptid, das von den genannten Tumoren aufgenommen wird.

Das Inkretinhormon Glucagon-Like Peptid-1 (GLP-1) sowie seine Analoga Exendin 3 und Exendin 4 (aus dem Speichel des Gilamonsters (Heloderma horridum und Heloderma suspectum)) sind Peptide, für die Insulinome und kleinzellige Bronchialkarzinome - neben vielen anderen Tumorarten - Rezeptoren exprimieren. Insulinome stammen von den insulinproduzierenden $\beta$-Zellen in den Langerhans'schen Inseln des Pankreas ab, in denen GLP-1 sowie Exendin 3 und Exendin 4 eine postprandiale Insulinsekretion auslösen.

**Stand der Technik**

Zur Verwendung von Glucagon-Like Peptide-1 (GLP-1) in der Szintigraphie ist eine Markierung der Peptide erforderlich. Die Verfahren dazu und zur Markierung von Proteinen mit Radionukliden sind dem Fachmann bekannt und in zahlreichen Patentschriften (z.B. DE 690 18 226 T2) und wissenschaftlichen Arbeiten belegt. Die dabei beschriebenen Peptide zur Anwendung in der diagnostischen Bildgebung und zur Vermittlung von therapeutisch wirksamen Molekülen in pathologisches Gewebe werden in der Regel am N-terminalen Ende über ein Amin in das Peptid eingeführt. Die Peptide müssen dabei bezüglich der Stabilisierung weiter modifiziert werden.

Das in der US 2003/0232761A1 verwendete GLP-1 und sein Derivat GLP-1 (7-37) sind beispielsweise am N-terminalen Ende über ein Amin modifiziert. Somit steht das N-terminale Ende von GLP-1 für die Bindung an einen GLP-1-Rezeptor nicht mehr zur Verfügung, so dass mit diesen Peptiden nur eine ungenügende Rezeptorbindung und Internalisierung möglich ist, diese Peptide also nicht zur Verwendung in der Radiopeptidtherapie von Insulinomen und kleinzelligen Bronchialkarzinomen geeignet sind. Eine Mutation z.B. ein Austausch einer Aminosäure innerhalb der Peptidsequenz von GLP-1 und Exendin-3 oder Exendin-4 und deren mögliche Modifikation durch ein Therapeutikum oder ein signalgebendes Molekül führt erfahrungsgemäß meist dazu, dass die Struktur des Peptides so gestört wird, dass keine Bindung an den Rezeptor mehr möglich ist.

Eine weitere Methode zur Modifikation von GLP-1, ohne Beeinflussung des N-Terminus ist derzeit nicht bekannt. Darüber hinaus sind keine GLP-1 Derivate bekannt, die markiert oder unmarkiert zum Einsatz in der Radiotherapie von Insulinomen und kleinzelligen Bronchialkarzinomen geeignet sind.

**Aufgabe**

Aufgabe der vorliegenden Erfindung ist daher, den Mangel im Stand der Technik zu beseitigen und Peptide bereitzustellen, die markiert werden können und auch mit dieser Markierung an den GLP-1-Rezeptor binden und die zur Herstellung eines Mittels für Diagnostik und Therapie von Erkrankungen, bei denen die GLP-1 Rezeptorexpression eine Rolle spielt, eingesetzt werden.

**Lösung der Aufgabe**

Diese Aufgabe wird erfindungsgemäß durch die Ansprüche gelöst, durch Peptidderivate von Exendin-4, die am C-Terminus über ein Amin modifiziert sind und über den N-Terminus an den GLP-1-Rezeptor binden, und die zur Aminosäuresequenz des Peptids

Exendin-4:

$$\text{H-}_1\text{His-}_2\text{Gly-}_3\text{Glu-}_4\text{Gly-}_5\text{Thr-}_6\text{Phe-}_7\text{Thr-}_8\text{Ser-}_9\text{Asp-}_{10}\text{Leu-}_{11}\text{Ser-}_{12}\text{Lys-}_{13}\text{Gln-}_{14}\text{Met-}_{15}\text{Glu-}_{16}\text{Glu-}_{17}\text{Glu-}_{18}\text{Ala-}_{19}\text{Val-}_{20}\text{Arg-}_{21}\text{Leu-}_{22}\text{-Phe-}_{23}\text{Ile-}_{24}\text{Glu-}_{25}\text{Trp-}_{26}\text{Leu-}_{27}\text{Lys-}_{28}\text{Asn-}_{29}\text{Gly-}_{30}\text{Gly-}_{31}\text{Pro-}_{32}\text{Ser-}_{33}\text{Ser-}_{34}\text{Gly-}_{35}\text{Ala-}_{36}\text{Pro-}_{37}\text{Pro-}_{38}\text{Pro-}_{39}\text{Ser-NH2}$$

eine Sequenzidentität von über 90% aufweisen, wobei die Peptidderivate eine Attachementgruppe A, die am C-Terminus lokalisiert ist und aus einem Amin besteht, aufweisen, wobei an die Attachementgruppe A ein Chelator zur komplexen Kopplung von Radionukliden gekoppelt ist, und wobei an den Chelator ein Radionuklid gekoppelt ist ausgewählt aus der Gruppe F-18, Cu-64, Cu-67, Ga-67, Ga-68, Y-86, Y-90, Tc-99m, In-111, I-123, I-124, I-131, Lu-177, Re-186, Re-188, Pt-193m, Pt-195m, Ac-225, At-211, Bi-213, Sm-153 oder Er-169.

Diese Peptidderivate werden markiert zur Herstellung eines Mittels zur Diagnostik und Therapie gut- und bösartiger Erkrankungen, bei denen die GLP-1 Rezeptorexpression eine Rolle spielt, eingesetzt.

Mit Hilfe dieser Peptidderivate von Exemdin-4, wird die Herstellung eines Mittels zur szintigraphischen Anwendung realisiert, das zur Diagnostik und Theraphie von GLP-1-Rezeptor exprimierenden Tumoren, darunter NET (insbesondere von Insulinomen) und kleinzelligen Bronchialkarzinomen eingesetzt wird.

Dies ist erstmals möglich, da die erfindungsgemäßen Peptidderivate über ein Amin am C-Terminus modifiziert sind, und so der N-Terminus für die Bindung an den GLP-1-Rezeptor zur Verfügung steht.

Durch die Bindung der erfindungsgemäßen radioaktiv markierten Peptidderivate an den GLP-1-Rezeptor ist die Darstellung von GLP-1-Rezeptor exprimierenden Tumoren möglich und damit erfolgt eine erhebliche Verbesserung der Patientenversorgung. In erster Linie sind NET gastroenteropankreatische NET, wie Insulinome, bei denen zur Zeit kein nicht-invasives Verfahren mit einer ausreichenden Sensitivität verfügbar ist oder im Bereich der Lunge lokalisierte kleinzellige Bronchialkarzinome, bei denen die spezifische Differenzierung zwischen entzündlichen Prozessen und Tumoren bzw. Metastasen ebenfalls derzeit durch kein nicht-invasives Verfahren möglich ist.

Weiterhin wird mittels der erfindungsgemäßen Peptidderivate die Dichte Insulin-produzierender Zellen im Pankreas dargestellt sowie die Expression von GLP-1-Rezeptoren *in vivo* und *in vitro*. Dies ist z.B. bei der Darstellung GLP-1-Rezeptoren exprimierender Zellen beim Diabestes mellitus eine *in vivo*-Darstellung, da dies die Zellen sind, die auch Insulin sezernieren. Die Darstellung der GLP-1-Rezeptordichte im Pankreas ist besonders bei Patienten mit Diabetes mellitus während und nach der Therapie mit Medikamenten wichtig. Zusätzlich wird die Verteilung von GLP-1-Rezeptoren in malignen und benignen Geweben dargestellt. Fragestellungen sind hier sowohl klinischer als auch wissenschaftlicher Natur, da es noch keine umfassenden *in-vivo* Daten zur GLP-1-Rezeptorverteilung beim Menschen gibt.

Die Erfindung hat also den Vorteil, dass Peptidderivate von Exendin-4 zur Herstellung eines Mittels insbesondere zur rezeptorgerichteten spezifischen Darstellung und Therapie insbesondere von NET, hier besonders von Insulinomen und kleinzelligen Bronchialkarzinomen eingesetzt werden.

Insbesondere bei der Diagnostik von kleinzelligen Bronchialkarzinomen ist eine GLP-1-Rezeptorszintigraphie anwendbar und erlaubt erstmals die spezifische Detektion von metastatisch befallenen Lymphknoten (entzündlich veränderte Lymphknoten versus befallene Lymphknoten).

Die Verwendung der erfindungsgemäßen Peptidderivate von Exendin-4 erfolgt weiterhin als Mittel zur Diagnostik und Therapie aller gut- und bösartiger Erkrankungen, bei denen die GLP-1 Rezeptorexpression eine Rolle spielt, im Besonderen als Kontrastmittel mit der Magnetresonanztomographie (MRT), als radioaktives Mittel im der Szintigraphie (SPECT, Single Photonen Computer Tomographie) bzw. Radiopetidtheraphie, in der PET (Positronen Emissions Tomographie), rezeptorvermittelten Chemotherapie und der optischen Diagnostik. Wobei unter optischer Diagnostik die Anregung eines fluoreszierenden Moleküls durch eine bestimmte Wellenlänge, welche eine nachfolgende Emission von Licht einer anderen Wellenlänge auslöst, verstanden wird. Dabei wird die emittierte Wellenlänge detektiert.

Es ist dem Fachmann leicht möglich die Art der Markierung am C-terminalen Terminus der Peptidderivate von Exendin-4 in Abhängigkeit von der gewünschten Anwendung auszuwählen, die z.B. für die Szintigraphie bzw. Radiotherapie aus radioaktiven Nukliden, für das Kontrastmittel in der Magnetresonanztomographie (MRT) beispielsweise aus Gadolinum, für endoskopische oder wissenschaftliche Untersuchungen aus Fluoreszenzfarbstoffen besteht.

Erfindungsgemäß werden unter malignen Erkrankungen bösartige Erkrankungen verstanden, bei denen die betroffenen Gewebe Veränderungen ihres Differenzierungsgrades gegenüber gesunden Geweben aufweisen, invasives Wachstum zeigen oder deren Gewebe sich über Blut- oder Lymphbahnen ausbreitet. Hierzu gehören alle neuroendokrinen Tumoren, insbesondere die des Gastrointestinaltraktes; insbesondere auch Insulinome, Bronchialkarzinome, Pankreaskarzinom und alle anderen bösartigen Erkrankungen, die mit einer Überexpression des GLP-1-Rezeptors verbunden sind.

Erfindungsgemäß werden unter benignen Erkrankungen gutartige Erkrankungen verstanden, die sich dadurch auszeichnen, dass die betroffenen Gewebe ihren Differenzierungsgrad nicht wesentlich verlieren, kein invasives Wachstum zeigen und keine Gewebeabsiedlungen über Lymph- oder Blutstrom bilden. Hierzu gehört z.B. Diabetes mellitus aber auch Störungen des Essverhaltens oder der Psyche.

**Charakterisierung der Peptidderivate**

Überraschenderweise wurde gefunden, dass Peptidderivate von Exendin-4, die am C-Terminus über Amin modifiziert sind, über einen N-Terminus an den GLP-1-Rezeptor binden. Sie zeigen sogar eine dem natürlichen Peptid ähnliche sehr hohe Affinität zum GLP-1-Rezeptor. Experimente mit tumortragenden Nacktmäusen zeigen eine spezifische Aufnahme in GLP-1-Rezeptor-positives Tumorgewebe.

Die erfindungsgemäßen Peptidderivate werden über einen Chelator am C-terminalen Amin markiert als Mittel zur Diagnostik und Therapie gut- und bösartiger Erkrankungen, bei denen die GLP-1 Rezeptorexpression eine Rolle spielt, eingesetzt. Die Art der Markierung besteht dabei insbesondere aus einem Radiometall.

Der Vorgang und die Verfahren zur Markierung sind dem Fachmann geläufig (z.B. DE 690 18 226 T2) und erfolgen durch Kopplung von Radionukliden, amagnetischen Metallen und andere MRT-Kontrastmitteln oder Fluoreszenzfarbstoffen, so dass die Rezeptorbindung oder Internalisierung der erfindungsgemäßen Peptidderivate nicht beeinträchtigt wird und der GLP-1-rezeptorbindende N-Terminus ungebunden bleibt.

Die Aminosäuresequenzen der Ausgangspeptide:

**Exendin-4:**

H-His-Gly-Glu-Gyl-Thr-Phe-Thr-Ser-Asp-Leu-Ser-Lys-Gln-Met-Glu-

1　　2　　3　　4　　5　　6　　7　　8　　9　　10　　11　　12　　13　14　　15

Glu-Glu-Ala-Val-Arg-Leu-Phe-Ile-Glu-Trp-Leu-Lys-Asn-Gly-Gly-

16　17　18　19　20　21　22　23　24　25　26　27　28　29　30

Pro-Ser-Ser-Gly-Ala-Pro-Pro-Pro-Ser-NH2

31　32　33　34　35　36　37　38　39

Erfindungsgemäß werden folgende Peptidderivate von Exendin-4 hergestellt:

**Exendin-4 (z-k)A$^{1-40}$**

Wobei gilt:

z = Aminosäuren 1-38 der Exendin-4 Aminosäuresequenz
k = Aminosäuren 2-39 der Exendin-4 Aminosäuresequenz,

wobei die Peptidderivate zur Aminosäuresequenz von Exendin-4 eine Sequenzidentität von über 90% aufweisen, und

A = Attachementgruppe bestehend aus einer oder mehreren Aminosäuren bzw. deren Derivate als Signalmolekül an sich oder zur Bindung von Signalmolekülen oder zur Stabilisierung. A ist am C-Terminus lokalisiert und ist ein Amin bevorzugt Lysin oder alternativ eine andere Aminosäure mit einem freien Amin wie z.B. Ornitin oder eine organischen Gruppe mit einem freien Amin an das ein Chelator zur Markierung mit Radionukliden gekoppelt ist. Chelatoren sind beispielsweise DTPA (Diethylenetriaminepentaacetic acid, alternativ einsetzbar N,N-Bis(2-[bis(carboxymethyl)amino]-ethyl)glycine), alternativ DOTA (1,4,7,10-Tetraazacyclododecane-1,4,7,10-tetra-acetic acid), HYNIC (6-Hydrazinopyridin-3-carbonsäure), MAG3 (mercaptoacetyl-glycylglycylglycine), N4 (1,4,8,11-tetraazaundecane) und die bekannten Derivate aller genannten Chelatoren.

Exendin-4(z-k)A$^{1-40}$

hierbei sind verschieden lange Exendin-4 Derivate umfasst, die zur Aminosäuresequenz von Exendin-4 eine Sequenzidentität von über 90% aufweisen, wobei z die Zahlen 1 bis 38 annehmen kann, aber kleiner ist als k, welches die Zahlen 2 bis 39 annehmen kann. A ist die Attachementgruppe, die am C-Terminus steht und um eins höher ist als y. Die Attachementgruppe ist bevorzugt das Amin Lysin.

Insbesondere ist bevorzugt das Peptidderivat:

MC 11: (DTPA-Lys$^{40}$) Exendin-4 amid

Die Synthese erfolgt z.B. über Firma Peptide Specialty Laboratories GmbH nach der Methode von Merrifield und Reinigung über HPLC.

MC 11 (DTPA-Lys$^{40}$) Exendin-4 amid besteht aus der gesamten Aminosäuresequenz von Exendin-4 und trägt am C-terminalen Ende als Aminosäure mit einem freien Amin vorzugsweise ein Lysin an Position 39 sowie den Chelator DTPA.

Dem Fachmann ist ersichtlich, dass somit verschieden lange Peptidderivate von Exendin-4 vorliegen können.

Die erfindungsgemäßen Peptidderivate von Exendin-4, die am C-Terminus über ein Amin modifiziert sind und über den N-Terminus an den GLP-1-Rezeptor binden, umfassen auch Moleküle, die sich von den Aminosäuresequenzen des beschriebenen Peptids Exendin-4 an einer oder mehreren Positionen unterscheiden und einen hohen Grad an Homologie zu dieser Sequenz aufweisen. Homologie meint dabei eine Sequenzidentität von über 90%. Die Abweichungen zu den oben beschriebenen Aminosäuresequenzen können dabei durch Deletion, Substitution und/oder Insertion entstanden sein.

**Markierung der Peptidderivate**

Die erfindungsgemäßen Peptidderivate werden zur Herstellung eines Mittels zur Diagnostik und Therapie gut- und bösartiger Erkrankungen, bei denen die GLP-1 Rezeptorexpression eine Rolle spielt, in einem geeigneten Stabilisierungspuffer gelöst, z.B. zur Stabilisierung der Metalle in vorzugsweise 0,5 M Natriumacetat pH 5,4 mit einer Konzentration von ca. $10^{-3}$ M. Alternativ wird zur Stabilisierung von Fluoreszenzfarbstoffen ein Puffer aus Ammoniumacetat bevorzugt, zur Stabilisierung von Chemotherapeutika und Kontrastmitteln ein physiologischer Puffer.

Die Markierung erfolgt erfindungsgemäß an der Attachementgruppe A durch Koppelung von Radionukliden. Dabei werden je nach Art der Anwendung für *in vivo* oder *in vitro* unterschiedliche Verfahren verwendet.

Als Radionuklide zur komplexen Kopplung werden verwendet:

| Nuklid | Anwendungsverfahren | $t_{1/2}$[h] | Emittierende Strahlung | Energie[keV] | Art der Kopplung |
|---|---|---|---|---|---|
| F-18 | PET | 1,8 | B$^+$ | 634 | Kovalent |
| Cu-64 | PET | 12,7 | B$^+$ | 1673 | Komplex |
| Cu-67 | Therapie | 61,8 | $\beta^-$ $\gamma$ | 391 184 | Komplex |
| Ga-67 | SPECT | 79,2 | $\gamma$ | 93/184/300 | Komplex |
| Ga-68 | PET | 1,1 | B$^+$ | 2921 | Komplex |
| Y-86 | PET | 14,8 | $\beta^+$ $\gamma$ | 1220 1076/1153 | Komplex |
| Y-90 | Therapie | 64,1 | B$^-$ | 2280 | Komplex |
| Tc-99m | SPECT | 6 | $\gamma$ | 140 | Komplex |
| In-111 | SPECT | 67,2 | $\gamma$ | 171/245 | Komplex |
| I-123 | SPECT | 13,2 | $\gamma$ | 158 | Kovalent |
| I-124 | PET | 101 | $\beta^+$ $\gamma$ | 2137/1534 602 | Kovalent |

(fortgesetzt)

| Nuklid | Anwendungsverfahren | $t_{1/2}$[h] | Emittierende Strahlung | Energie[keV] | Art der Kopplung |
|--------|---------------------|--------------|------------------------|--------------|------------------|
| I-131 | Therapie | 192 | γ β⁻ | 364 606 | Kovalent |
| Lu-177 | Therapie | 158 | γ β⁻ | 208 112/208 | Komplex |
| Re-186 | Therapie | 88,8 | γ β⁻ | 137 1071 | Komplex |
| Re-188 | Therapie | 17 | γ β⁻ | 155/477/632 1965/2120 | Komplex |
| Pt-193m | Therapie | 104 | γ Auger e⁻ | 135 | Komplex |
| Pt-195m | Therapie | 96 | Y Auger e⁻ | 98 | Komplex |
| Ac-225 | Therapie | 240 | γ α | 99, 150 | Komplex |
| At-211 | Therapie | 7,2 | γ Auger e⁻ | 687 | Komplex kovalent |
| Bi-213 | Therapie | 0,76 | γ α | 440 | Komplex |
| Sm-153 | Therapie | 46 | γ β⁻ | 103 | komplex |
| Er-169 | Therapie | 226 | B⁻ | 100 | komplex |

PET (Positronen Emissions Tomographie), SPECT (Single Photonen Computer Tomographie)

Chelatoren sind beispielsweise DTPA (Diethylenetriaminepentaacetic acid, N,N-Bis(2-[bis(carboxymethyl)amino]-ethyl)glycine), DOTA (1,4,7,10-Tetraazacyclododecane-1,4,7,10-tetra-acetic acid), HYNIC (6-Hydrazinopyridin-3-carbonsäure), MAG3 (mercaptoacetyl-glycylglycylglycine), N4 (1,4,8,11-tetraazaundecane) oder die Derivate aller genannten Chelatoren.

Je nach Art der Anwendung der erfindungsgemäßen Proteinderivate und dem aus ihnen hergestellten Mittel zur Diagnostik und Therapie gut- und bösartiger Erkrankungen, bei denen die GLP-1 Rezeptorexpression eine Rolle spielt, wird die Markierungsreaktion in zwei Varianten durchgeführt.

**Markierung zur *in vitro* Anwendung in der Radiotherapie**

3 μL der in einem geeigneten Stabilisierungspuffer vorzugsweise 0,5 M Natrium-acetat pH 5,4 mit einer Konzentration von ca. $10^{-3}$ M gelösten erfindungsgemäßen Peptidderivate werden zur Markierung zu 500 μL 0,5M Natriumacetat pH 5,4 gegeben. Der pH-Wert liegt dabei zwischen 3-6. Dazu wird 185 MBq $^{111}$InCl$_3$ (Tyco, Petten, The Netherlands) in 0,1 M HCl 500 μL hinzugefügt und während 30 Minuten bei 37°C inkubiert. Um alle Bindungsstellen abzusättigen wird nochmals 3 μL $10^{-3}$ M Lösung $^{nat}$InCl$_3$ zugegeben und nochmals 30 min inkubiert.

Die Qualitätskontrolle wird über eine HPLC- Säule vorgenommen:

Säule: CC 250/4.6 Nucleosil 120-5 C18 (Machery-Nagel, Oenisingen, Schweiz) Gradient: 0->5 min 100% 0.05 M NH$_4$OOCCH$_3$, pH 5.4 (Puffer A); 5->25 min 100% Puffer A ->50% Puffer A/50% Acetonitril.

Die Qualitätskontrolle für eine in vitro Anwendung wird erfüllt bei einer Markierungsausbeute von mehr als 98%.

Somit steht ein radioaktiv markiertes Mittel zur Diagnostik und Therapie gut-und bösartiger Erkrankungen, bei denen die GLP-1 Rezeptorexpression eine Rolle spielt, zur Verfügung, dass beispielsweise in der Zell- und Gewebekultur mit Pankreaszellen eingesetzt wird.

**Markierung zur *in vivo* Anwendung in der Radiotherapie**

3 μL der in einem geeigneten Stabilisierungspuffer vorzugsweise 0,5 M Natriumacetat pH 5,4 mit einer Konzentration von ca. $10^{-3}$ M gelösten erfindungsgemäßen Peptidderivats werde zur Markierung zu 500 μL 0,5 M Natriumacetat pH 5,4 gegeben. Am Ende wird dazu 185 MBq $^{111}$InCl$_3$ (Tyco, Petten, The Netherlands) in 0,1 M HCl 500 μL dazugegeben und während 30 Minuten bei 37°C inkubiert. Die Qualitätskontrolle wird über eine HPLC-Säule vorgenommen:

Säule: CC 250/4.6 Nucleosil 120-5 C18 (Machery-Nagel, Oenisingen, Schweiz) Gradient: 0->5 min 100% 0.05 M $NH_4OOCCH_3$, pH 5.4 (Puffer A); 5->25 min 100% Puffer A ->50% Puffer A/50% Acetonitril.

Die Qualitätskontrolle für eine in vivo Anwendung wird erfüllt bei einer Markierungsausbeute von mehr als 98%.

Somit steht ein radioaktiv markiertes Mittel zur Diagnostik und Therapie gut-und bösartiger Erkrankungen, bei denen die GLP-1 Rezeptorexpression eine Rolle spielt zur Verfügung, dass beispielsweise zur Tumordetektion bei Patienten eingesetzt wird.

Der Begriff Patient bezieht sich dabei gleichermaßen auf Menschen und Wirbeltiere. Damit wird das Mittel in der Human- und Veterinärmedizin verwendet. Das therapeutisch und diagnostisch wirksame Mittel der vorliegenden Erfindung wird den Patienten, als Teil einer pharmazeutisch akzeptablen Komposition entweder oral, rektal, parenteral intravenös /intrararteriell, intramuskulär, subkutan, intrathekal, intracistemal, intracraniell, intravaginal, intraperitoneal, intravasculär, lokal (Puder, Salbe oder Tropfen) oder in Sprayform verabreicht. Die entsprechende Dosierung ist je nach Anwendungsfall für Diagnostik und Therapie gut- und bösartiger Erkrankungen, bei denen die GLP-1 Rezeptorexpression eine Rolle spielt, vom Arzt festzulegen.

**Internalisierungsstudie**

Die Internalisierungsstudie zeigt beispielhaft den Transport der über in vitro-Markierung radioaktiv markierten erfindungsgemäßen Peptidderivate in die Zelle.

In einer 6-well Platte werden 100 000 GLP-1-Rezeptor transfizierte CHO Zellen ausgesät. Die Zellen wachsen bis sie konfluent sind. Es werden 4 Gruppen gebildet:

Gruppe 1: totale Bindung, PBS gewaschen

Es werden 100 000 cpm $^{111}$In ($10^{-15}$ Mol) markiertes Peptid in 2 mL Medium zugegeben und 1 h bei 37°C inkubiert. Danach wird 3x mit PBS gewaschen und die Zellen mit 20 mM MOPS (3-Morpholinopropanesulfonic-acid) + 0,1% Triton-X-100 (pH 7,4) abgelöst. Die Aufnahme (Uptake) in die Zellen wird im γ-Counter gemessen. Die Zellzahl wird über den Proteingehalt mit dem Proteinassay-Kit von Bio-Rad (München, Deutschland) basierend auf der Methode von Bradford gemessen. Das Resultat wird in cpm/μg Protein angegeben.

Gruppe 2: unspezifische Bindung, PBS gewaschen

Es werden 20 μL einer $10^{-3}$ M GLP-1 Lösung und 100 000 cpm $^{111}$In markiertes Peptid in 2 mL Medium zugegeben und 1 h bei 37°C inkubiert. Danach wird 3x mit PBS gewaschen und die Zellen mit 20 mM MOPS (3-Morpholinopropansulfonylsäure) + 0,1% Triton-X-100 (pH 7,4) abgelöst. Der Uptake in den Zellen wird im γ-Counter gemessen. Die Zellzahl wird über der Proteingehalt mit dem Proteinassay-Kit von Bio-Rad (München, Deutschland) basierend auf der Methode von Bradford gemessen. Das Resultat wird in cpm/μg Protein angegeben.

Gruppe 3: totale Bindung, Säure gewaschen

Es werden 20 μL einer $10^{-3}$ M GLP-1 Lösung und 100 000 cpm $^{111}$In markiertes Peptid in 2 mL Medium zugegeben und 1 h bei 37°C inkubiert. Danach wird 1 x mit 0,1 M Natrium Acetatpuffer pH 4 gewaschen und 2x mit PBS gewaschen und die Zellen mit 20 mM MOPS (3-Morpholinopropansulfonylsäure) + 0,1% Triton-X-100 (pH 7,4) abgelöst. Der Uptake in den Zellen wird im γ-Counter gemessen. Die Zellzahl wird über den Proteingehalt mit dem Proteinassay-Kit von Bio-Rad (München, Deutschland) basierend auf der Methode von Bradford gemessen. Das Resultat wird in cpm/μg Protein angegeben.

Gruppe 4: unspezifische Bindung, Säure gewaschen

Es werden 20 μL einer $10^{-4}$ M GLP-1 Lösung und 100 000 cpm $^{111}$In markiertes Peptid in 2 mL Medium zugegeben und 1 h bei 37°C inkubiert. Danach wird 1 x mit 0,1 M Natrium Acetatpuffer pH 4 gewaschen und 2x mit PBS gewaschen und die Zellen mit 20 mM MOPS (3-Morpholinopropansulfonylsäure) + 0,1% Triton-X-100 (pH 7,4) abgelöst. Der Uptake in den Zellen wird im γ-Counter gemessen. Die Zellzahl wird über den Proteingehalt mit dem Proteinassay-Kit von Bio-Rad (München, Deutschland) basierend auf der Methode von Bradford gemessen. Das Resultat wird in cpm/μg Protein angegeben.

## Auswertung: *%IDsB = [(Res.3-Res.4)/(Res.1-Res.2)]\*100*

%IdsB = %Internalisierung der spezifischen Bindung

|  | %IdsB |
|---|---|
| MC11 | $70\pm7$ |

Das Ergebnis zeigt, dass ein guter Transport in die Zellen erfolgt.

**Bindungsstudien**

Bindungsstudien zeigen die spezifische Bindung der über in vivo-Markierung radioaktiv markierten erfindungsgemäßen Peptidderivate an den GLP-1 - Rezeptor.

In einer 6-well Platte werden 100 000 GLP-1-Rezeptor transfizierte CHO Zellen ausgesät. Die Zellen wachsen bis sie konfluent sind. Es werden dann in 2 mL 100 000 cpm $^{111}$In markiertes Peptid zugegeben. Um die Bindung zu testen wird mit 20 $\mu$L einer $10^{-3}$ M GLP-1 Lösung geblockt.

|  | %Blockierung |
|---|---|
| MC11 | $85\pm3$ |

Die *in vivo* Bioverteilung wird beispielsweise mit Nagern z.B. mit Nacktmäusen dargestellt. Dazu werden GLP-1 transfizierte CHO Zellen in Nacktmäuse injiziert. Nach ca. 3-5 Wochen sind Tumore einer Größe von ca. 300 mg gewachsen. Den Mäusen wird eines der erfindungsgemäßen 37 MBq $^{111}$In markierten Peptide über die Schwanzvene injiziert und die Mäuse werden unter der einer $\gamma$-Kamera nach 4 h gemessen.

Dabei erfolgt eine schnelle Clearance über die Niere und eine Aufnahme in der Niere. Eine hoher Aufnahme erfolgt ebenfalls im GLP-1 Rezeptor positivem Tumor, während ein GLP-1 Rezeptor negativer Tumor kaum eine Aufnahme zeigt. Eine leichte Aufnahme ist ebenfalls im Pankreas zu sehen, andere Organe zeigen keine sichtbare Aufnahme.

Man erkennt, dass Peptidderivate von Exendin-4 am C-Terminus über ein Amin modifiziert sind und über den N-Terminus an den GLP-1-Rezeptor binden, wobei sie zur Aminosäuresequenz des folgenden Peptids eine Sequenzidentität von über 90% aufweisen :

Exendin-4:

$$H\text{-}_1His\text{-}_2Gly\text{-}_3Glu\text{-}_4Gly\text{-}_5Thr\text{-}_6Phe\text{-}_7Thr\text{-}_8Ser\text{-}_9Asp\text{-}_{10}Leu\text{-}_{11}Ser\text{-}_{12}Lys\text{-}_{13}Gln\text{-}$$

$$_{14}Met\text{-}_{15}Glu\text{-}_{16}Glu\text{-}_{17}Glu\text{-}_{18}Ala\text{-}_{19}Val\text{-}_{20}Arg\text{-}_{21}Leu\text{-}_{22}\text{-}Phe\text{-}_{23}Ile\text{-}_{24}Glu\text{-}_{25}Trp\text{-}$$

$$_{26}Leu\text{-}_{27}Lys\text{-}_{28}Asn\text{-}_{29}Gly\text{-}_{30}Gly\text{-}_{31}Pro\text{-}_{32}Ser\text{-}_{33}Ser\text{-}_{34}Gly\text{-}_{35}Ala\text{-}_{36}Pro\text{-}_{37}Pro\text{-}$$

$$_{38}Pro\text{-}_{39}Ser\text{-}NH2$$

und wobei die Peptidderivate eine Attachmentgruppe A aufweisen.

Die Attachementgruppe A ist am C-Terminus lokalisiert ist und stellt ein Amin dar. Bevorzugt ist die Attachementgruppe A Lysin und/oder die Attachementgruppe A ist eine Aminosäure mit einem freien Amin wie Ornithin ist oder eine organische Gruppe mit einem freien Amin.

An die Attachementgruppe A ist ein Chelator zur Markierung mit Radionukliden gekoppelt.

Der Chelator ist ausgewählt aus der Gruppe N,N-Bis(2-[bis(carboxymethyl)amino]-ethyl)glycine), DOTA (1,4,7,10-Tetraazacyclododecane-1,4,7,10-tetra-acetic acid), HYNIC (6-Hydrazinopyridin-3-carbonsäure), MAG3 (mercaptoacetyl-glycylglycylglycine), N4 (1,4,8,11-tetraazaundecane) und deren bekannte Derivate, vorzugsweise DTPA (Diethylenetriamine-pentaacetic acid).

Die Markierung ist eine Koppelung von einem Radionuklid.

Das Radionuklid ist ausgewählt insbesondere aus der Gruppe F-18, Cu-64, Ga-67, Ga-68, Y-86, Y-90, Tc-99m, In-111, I-123, I-124,-I-131, Lu-177, Re-186, Re-188, Pt-193m, Pt-195m, Ac-225, At-211, Bi-213, Sm-153 oder Er-169.

Die Markierung erfolgt durch Koppelung von Radionukliden für in-vitro-Anwendungen durch Absättigung der Bindungsstellen mit $^{nat}InCl_3$.

Die Peptidderivate von Exendin-4 können zur Herstellung eines Mittels zur Diagnostik und Therapie gut- und bösartiger Erkrankungen verwendet werden, bei denen die GLP-1 Rezeptorexpression eine Rolle spielt. Die Peptidderivate von Exendin-4 können zur Bestimmung der Dichte Insulin-produzierender Zellen in einem Gewebe verwendet werden. Die Peptidderivate von Exendin-4 können zur Bestimmung der Expression von GLP-1 -Rezeptoren oder deren Dichte verwendet werden.

Man erkennt, dass ein Mittel zur Diagnostik und Therapie gut- und bösartiger Erkrankungen, bei denen die GLP-1 Rezeptorexpression eine Rolle spielt vorteilhaft markierte Peptidderivate von Exendin-4 enthalten kann. Weiter kann bei einem Mittel zur Diagnostik und Therapie gut- und bösartiger Erkrankungen, bei denen die GLP-1 Rezeptorexpression eine Rolle spielt, und welches markierte Peptidderivate von Exendin-4 enthält, die Markierung eine Koppelung von Radionukliden, MRT Kontrastmitteln, Fluoreszenzfarbstoffen und Chemotherapeutikum beinhalten.

Man erkennt dass ein Mittel, welches markierte Peptidderivate von Exendin-4 beinhaltet, zur Diagnostik und Therapie gut- und bösartiger Erkrankungen, bei denen die GLP-1 Rezeptorexpression eine Rolle spielt, verwendet werden kann. Man erkennt weiter, dass ein solches Mittel zur Diagnostik und Therapie, neuroendokriner Tumoren (NET) insbesondere Insulinomen und kleinzelligen Bronchialkarzinomen verwendet werden kann. Weiterhin kann ein Mittel, welches markierte Peptidderivate von Exendin-4 enthält, in der Szintigraphie, PET, SPECT, MRT, optischen Diagnostik, rezeptorvermittelten Chemotherapie, rezeptorvermittelten, zytostatischen oder zytotoxischen Therapie und Radiopeptidtherapie verwendet werden.

**Patentansprüche**

1. Peptidderivate von Exendin-4, die am C-Terminus über ein Amin modifiziert sind und über den N-Terminus an den GLP-1-Rezeptor binden, und die zur Aminosäuresequenz des Peptids Exendin-4:

$$H-_1His-_2Gly-_3Glu-_4Gly-_5Thr-_6Phe-_7Thr-_8Ser-_9Asp-_{10}Leu-_{11}Ser-_{12}Lys-_{13}Gln-_{14}Met-_{15}Glu-$$
$$_{16}Glu-_{17}Glu-_{18}Ala-_{19}Val-_{20}Arg-_{21}Leu-_{22}-Phe-_{23}Ile-_{24}Glu-_{25}Trp-_{26}Leu-_{27}Lys-_{28}Asn-_{29}Gly-$$
$$_{30}Gly-_{31}Pro-_{32}Ser-_{33}Ser-_{34}Gly-_{35}Ala-_{36}Pro-_{37}Pro-_{38}Pro-_{39}Ser-NH2$$

eine Sequenzidentität von über 90% aufweisen, wobei die Peptidderivate eine Attachementgruppe A, die am C-Terminus lokalisiert ist und aus einem Amin besteht, aufweisen, **dadurch gekennzeichnet, dass** an die Attachementgruppe A ein Chelator zur komplexen Kopplung von Radionukliden gekoppelt ist, wobei an den Chelator ein Radionuklid gekoppelt ist ausgewählt aus der Gruppe F-18, Cu-64, Cu-67, Ga-67, Ga-68, Y-86, Y-90, Tc-99m, In-111, I-123, I-124, I-131, Lu-177, Re-186, Re-188, Pt-193m, Pt-195m, Ac-225, At-211, Bi-213, Sm-153 oder Er-169.

2. Peptidderivate von Exendin-4 gemäß Anspruch 1 **dadurch gekennzeichnet, dass** die Attachementgruppe A bevorzugt Lysin ist.

3. Peptidderivate von Exendin-4 gemäß einem der Ansprüche 1 bis 2, **dadurch gekennzeichnet, dass** der Chelator ausgewählt ist aus der Gruppe DTPA (Diethylenetriamine-pentaacetic acid), N,N-Bis(2-[bis(carboxymethyl)amino]-ethyl)glycine), DOTA (1,4,7,10-Tetraazacyclododecane-1,4,7,10-tetra-acetic acid), HYNIC (6-Hydrazinopyridin-3-carbonsäure), MAG3 (mercaptoacetyl-glycylglycylglycine), N4 (1,4,8,11-tetraazaundecane) und deren bekannte Derivate.

4. Verwendung der Peptidderivate von Exendin-4 gemäß der vorangegangenen Ansprüche zur Herstellung eines Mittels zur Diagnostik und Therapie gut- und bösartiger Erkrankungen, bei denen die GLP-1 Rezeptorexpression eine Rolle spielt.

5. Verwendung der Peptidderivate von Exendin-4 gemäß der Ansprüche 1 bis 3 zur Herstellung eines Mittels zur rezeptorgerichteten spezifischen Darstellung und Therapie von neuroendokrinen Tumoren (NET) insbesondere Insulinomen und kleinzelligen Bronchialkarzinomen.

**6.** Mittel zur Diagnostik und Therapie gut- und bösartiger Erkrankungen, bei denen die GLP-1 Rezeptorexpression eine Rolle spielt, **dadurch gekennzeichnet, dass** es markierte Peptidderivate von Exendin-4 gemäß Anspruch 1 enthält.

**7.** Mittel gemäß Anspruch 6, wobei es sich bei der Diagnostik um die Szintigraphie handelt.

**8.** Mittel gemäß Anspruch 7, wobei es sich bei der Szintigraphie um die PET oder SPECT handelt.

**Claims**

**1.** Peptide derivates from exendin-4, **characterized by** a modification at the C-terminus by an amino and a bonding of the N-terminus to the GLP-1 receptor and the amino acid sequences of the peptide exendin-4:

H-His-Gly-Glu-Gly-Thr-Phe-Thr-Ser-Asp-Leu-Ser-Lys-Gln-Met-Glu-Glu-Glu-

1   2   3   4   5   6   7   8   9   10  11  12  13  14  15 16 17

Ala-Val-Arg-Leu-Phe-Ile-Glu-Trp-Leu-Lys-Asn-Gly-Gly-Pro-Ser-Ser-Gly-Ala-

18  19  20  21  22  23 24  25  26  27  28  29  30  31  32  33  34  35

Pro-Pro-Pro-Ser-NH$_2$

36  37  38  39

contains a sequence identity of more than 90 %, whereby the peptide derivates have an attachment group A, located at the C-terminus consisting of one amino, **characterized by** the fact that the attachment group A is a chelator for complex coupling is coupled to radionuclides, whereby a chelator is coupled onto the radionuclides chosen in particular from the group F-18, Cu-64, Cu-67, Ga-67, Ga-68, Y-86, Y-90, Tc-99m, In-111, I-123, I-124, I-131, Lu-177, Re-186, Re-188, Pt-193m, Pt-195m, Ac-225, At-211, Bi-213, Sm-153 or Er-169.

**2.** Peptide derivates from exendin-4 according to claim 1, **characterized by** the fact that the attachment group is preferably lysine.

**3.** Peptide derivates from exendin-4 according to one of the claims 1 to 2, **characterized by** the fact that the chosen chelator is in particular from the group DTPA (Diethylenetriaminepentaacetic acid), N,N-Bis(2-[bis(carboxymethyl) amino] -ethyl) glycine), DOTA (1,4,7,10-Tetraazacyclodo-decane-1,4,7,10-tetra-acetic acid), HYNIC (6-Hydrazinopyridin-3-carbonic acid), MAG3 (mercaptoacetyl-glycylglycylglycine), N4 (1,4,8,11-tetraazaundecane) and all known derivatives.

**4.** Application of peptide derivates from exendin-4 according to the above mentioned claims for the production of an agent for diagnostics and therapy of benign and malignant diseases, in which GLP-1 receptor expression plays a role.

**5.** Application of peptide derivates from exendin-4 according to the claim 1 to 3 for the production of an agent for the receptor-targeted specific representation and therapy of neuroendocrine tumors (NET), particularly insulinomas and small-cell bronchial carcinomas.

**6.** Agent for the diagnostic and therapy of benign and malignant diseases, in which GLP-1 receptor expression plays a role, **characterized by** the fact that it contains labeled peptide derivates from exendin-4, according to claim 1.

**7.** Agent according to claim 6, whereat the diagnostic is realized as scintigraphy.

**8.** Agent according to claim 7, whereat the scintigraphy is realized in PET or SPECT.

## Revendications

1. Dérivés peptidiques de l'exendine-4, qui sont modifiés sur le C-terminal par le biais d'une amine et qui se lient au récepteur GLP-1 par le biais du N-terminal et qui, pour la séquence d'acides aminés du peptide exendine-4 :

$$H\text{-}_1His\text{-}_2Gly\text{-}_3Glu\text{-}_4Gly\text{-}_5Thr\text{-}_6Phe\text{-}_7Thr\text{-}_8Ser\text{-}_9Asp\text{-}_{10}Leu\text{-}_{11}Ser\text{-}_{12}Lys\text{-}_{13}Gln\text{-}_{14}Met\text{-}_{15}Glu\text{-}_{16}Glu\text{-}_{17}Glu\text{-}_{18}Ala\text{-}_{19}Val\text{-}_{20}Arg\text{-}_{21}Leu\text{-}_{22}Phe\text{-}_{23}Ile\text{-}_{24}Glu\text{-}_{25}Trp\text{-}_{26}Leu\text{-}_{27}Lys\text{-}_{28}Asn\text{-}_{29}Gly\text{-}_{30}Gly\text{-}_{31}Pro\text{-}_{32}Ser\text{-}_{33}Ser\text{-}_{34}Gly\text{-}_{35}Ala\text{-}_{36}Pro\text{-}_{37}Pro\text{-}_{38}Pro\text{-}_{39}Ser\text{-}NH2$$

présentent une identité de séquence de plus de 90 %, les dérivés peptidiques présentant un groupe d'attache A qui est localisé sur le C-terminal et se compose d'une amine, **caractérisés en ce qu'**un agent chélatant est couplé au groupe d'attache A pour le couplage complexe de radionucléides, un radionucléide étant couplé à l'agent chélatant et étant choisi dans le groupe F-18, Cu-64, Cu-67, Ga-67, Ga-68, Y-86, Y-90, Tc-99m, In-111, I-123, I-124, I-131, Lu-177, Re-186, Re-188, Pt-193m, Pt-195m, Ac-225, At-211, Bi-213, Sm-153 ou Er-169.

2. Dérivés peptidiques de l'exendine-4 selon la revendication 1, **caractérisés en ce que** le groupe d'attache A est de préférence la lysine.

3. Dérivés peptidiques de l'exendine-4 selon l'une des revendications 1 à 2, **caractérisés en ce que** l'agent chélatant est sélectionné dans le groupe DTPA (acide diéthylènetriamine-pentaacétique), N,N-bis(2-[bis(carboxyméthyl)amino]-éthyl)glycine), DOTA (acide 1,4,7,10-tétraazacyclododécane-1,4,7,10-tétraacétique), HYNIC (acide 6-hydrazinopyridine-3-carboxylique), MAG3 (mercaptoacétyl-glycylglycylglycine), N4 (1,4,8,11-tétraazaundécane) et de leurs dérivés connus.

4. Utilisation des dérivés peptidiques de l'exendine-4 selon les revendications précédentes, pour la fabrication d'un moyen de diagnostic et de thérapie de maladies bénignes et malignes, dans lesquelles l'expression du récepteur GLP-1 joue un certain rôle.

5. Utilisation des dérivés peptidiques de l'exendine-4 selon les revendications 1 à 3, pour la fabrication d'un moyen de représentation spécifique ciblant le récepteur et de thérapie de tumeurs neuroendocrines (NET), en particulier d'insulinomes et de carcinomes bronchiques microcellulaires.

6. Moyen de diagnostic et de thérapie de maladies bénignes et malignes, dans lesquelles l'expression du récepteur GLP-1 joue un certain rôle, **caractérisé en ce qu'**il contient des dérivés peptidiques marqués par l'exendine-4 selon la revendication 1.

7. Moyen selon la revendication 6, dans lequel, concernant le diagnostic, il s'agit de la scintigraphie.

8. Moyen selon la revendication 7, dans lequel, concernant la scintigraphie, il s'agit de PET ou de SPECT.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69018226 T2
- US 20030232761 A1